# EUROPEAN PATENT SPECIFICATION

(11) **EP 0 171 506 B1**
(45) Date of publication and mention of the grant of the patent: **22.09.1993**
(21) Application number: 85103962.8
(22) Date of filing: 11.05.1983
(51) Int. Cl.: A61L 2/04, C12N 7/04, A61K 35/16, A61K 39/21, A61K 39/245, A61K 39/29

(54) **A method for the heat treatment of plasma or plasma fractions and compositions obtained thereby**
Verfahren zur Wärmebehandlung von Plasma oder Plasmafraktionen und so erhaltene Zusammensetzungen
Procédé de traitement du plasma ou de fractions du plasma par la chaleur et compositions ainsi obtenues

(30) Priority: 13.05.1982 US 377863
(43) Date of publication of application: 19.02.1986
(62) Divisional of application: 83104642.0
(73) Proprietor: Cedars-Sinai Medical Center, Los Angeles, California 90048-0750 (US)
(72) Inventor: Rubenstein, Alan, Beverly Hills, Calif. 90210 (US)
(74) Representative: Hansen, Bernd, Dr. Dipl.-Chem.

(56) References cited:
- WO-A-82/03871
- FR-A- 1 588 829
- US-A- 3 041 242
- PROCEEDINGS OF THE XIV CONGRESS OF THE WORLD FEDERATION OF HEMOPHILIA, 3er-7th July 1981; A. RUBINSTEIN: "Heated lyophilized factor VIII and factor IX concentrate"
- BLOOD, vol. 58, no. 5, 1st September 1981, Washington, US; A. RUBINSTEIN: "heated lyophilized factor VIII concentrate and factor IX concentrate studies in Hemophilia A and B dogs"
- XII INTERNATINAL CONGRESS ON BLOOD TRANSFUSION ABSTRACTS, 17th-23rd August 1969, pages 473-474, Publis. "MIR", Moscow, USSR; G.Y. ROZENBERG et al.: "Thermoinactivation of virus of Botkin's disease (Hepatite Virus) in dry fibrinogen and albumin preparations"
- CHEMICAL ABSTRACTS, vol. 89, no. 9, August 28, 1978, page 278, abstract no. 73715j, Columbus, Ohio, US; E. GONCZOL et al.: "The effect of heat-inactivated murine cytomegalovirus on host DNA synthesis of different cells"; & J. Gen. Virol. 1978, 39(3), 415-26

## Description

The present invention relates generally to heat treated plasma. In particular the invention is directed to heat treated fractions which may be heated in lyophilized form for the purpose of inactivating undesirable microorganisms and viruses, such as cytomegalovirus, Epstein-Barr virus and hepatitis virus present in the fractions.

Utilization of clotting factor concentrates obtained from fractionated blood plasma for the purpose of intervening therapeutically with respect to inherited bleeding disorders such as hemophilia is severely compromised as a consequence of the inordinate risk posed to the hemophiliac patient by the presence of hepatitis virus in the concentrates. For example, commercial Factor VIII and IX concentrates are typically employed to increase the clotting ability of a hemophilia victim's blood, but these concentrates are prepared from pools of plasma contributed by thousands of donors and contain the inherent hepatitis risk of a like number of single unit transfusions. As McCullen and Zuckerman have shown, see Journal of Medical Virology, Vol. 8, No. 29 (1981), despite stringent screening of individual donors for hepatitis B surface antigens (HBsAg), such plasma pools clearly transmit both hepatitis B and non-A, non-B hepatitis.

Hepatitis transmission by albumin and other heat-stable plasma components unrelated to blood coagulation has heretofore been prevented by heating the plasma components in solution at temperatures of 60° C. for ten hours. Similar attempts to heat clotting factor concentrates in solution, by way of contrast, have been shown to markedly reduce or eliminate clotting factor activity in the concentrates and thus do not appear to offer a viable solution to the problem of hepatitis transmission associated with conventional hemophiliac therapy. More recently, highly purified Factor VIII precipitate has been dissolved in a solution of sucrose glycine and heated for ten hours at 60° C. Although the Factor VIII concentrate subsequently derived from the heated precipitate does retain clotting factor activity, the yields obtained using this approach are very low, e.g., about 8%. See Heimburger, et al., Hemostatis, Vol. 10 (supplement 1), p. 204 (1981) and Heimburger, et al., Blut, Vol. 44, p. 249-251 (1982).

As a net result, the prior art to date does not furnish any means for effectively inactivating hepatitis virus present in clotting factor concentrates nor does the prior art teach a means for effectively inactivating hepatitis virus present in clotting factor concentrates nor does the prior art teach a means for preventing the transmission of hepatitis virus to patients undergoing therapy with clotting factor concentrates.

According to the present invention the heat treating of substantially dry compositions including factor VIII inactivates, reduces or eliminates the infectivity of the microorganisms, such as viruses present therein without reducing clotting factor activity.

Also by the present invention is included the heat treating of clotting factor concentrates containing Factor VIII wherein the concentrates are first prepared in lyophilized form to enhance the stability of the concentrates during the heating process.

Heat-treated lyophilized plasma fractions preferably produce a composition and a vaccine effective against both hepatitis B virus and non-A, non-B hepatitis virus.

These and other aspects of the present invention are achieved by lyophilizing either whole plasma or plasma fractions including Factor VIII concentrate and thereafter subjecting the lyopholized whole plasma or plasma fractions to elevated temperatures for varying periods of time.

The ability to isolate clotting factors present in human blood has been indispensable in understanding the pathology of hemophilia and other inherited bleeding disorders. Concommitantly, the discovery of plasma fractionation schemes for obtaining practical quantities of clotting factor concentrates has enabled medical science to utilize the clotting factor concentrates as therapeutic tools in treating bleeding disorders. Transfusion therapy employing Factor VIII and Factor IX concentrates in particular has proven quite successful in ministering to hemophiliac patients. Unfortunately, the risk of hepatitis transmission due to the large number of plasma donors required for commercial production of clotting factor concentrates remains as the one serious drawback associated with transfusion therapy. A typical plasma fractionation scheme, disclosed in Seminars in Thrombosis and Hemostatis, Vol. VI, No. 1, p. 4 (1979), yields cryoprecipitate and supernate, the former fraction constituting a source of both Factor VIII concentrate and fibrinogen and the latter fraction constituting a source of Factor IX concentrate in addition to Factors II, VIII, and I concentrates. As Gerety and Eyster have demonstrated in "Hepatitis Among Hemophiliacs", Non-A, Non-B Hepatitis, p.103-106 (1981), hepatitis B virus initially present in whole plasma is distributed to the Factor VIII and Factor IX derivatives during the plasma fractionation process. As also demonstrated by Maynard and Bradley, "Transmission by Blood Products", Non-A Non-B Hepatitis, p. 78-79 (1981), non-A, non-B hepatitis exists in both Factor VIII and Factor IX derivatives. Previous attempts to heat-treat clotting factor concentrates in solution for the purpose of inactivating hepatitis virus have been ineffective. The development of techniques for lyophilizing clotting factor derivatives, however, has opened a new avenue of exploration with regard to stabilizing clotting factor derivatives during the heat treating process, in turn establishing a means for inactivating hepatitis virus present in the clotting factor derivatives without destroying clotting factor activity.

### Test Procedures for Verifying Retention of Clotting Factor Activity

Paired samples of various lyophilized plasma fractions, each such pair having identifcal lot numbers, were received from several manufacturers. The samples generally weighed less than 100 g and were packaged in vials having volumes of 60 ml to 90 ml. One sample in each pair was heated, either by placing the sample vial in a water bath or dry oven at a predetermined temperature under room pressure for a predetermined period of time, or by placing the lyophilized material itself in a dry oven without the vial present. The remaining sample in each pair served as a control and was refrigerated at 4°C - 6°C during the heat-treating process. Following heat treatment, both the control and heat-treated lyophilized samples were reconstituted with sterile water. Reconstitution was generally carried out according to manufacturer's specifications, although the solubility of some heat-treated samples was markedly improved by increasing the amount of sterile water used during reconstitution over that recommended by the manufacturer. In vitro Factor VIII and Factor IX assays were performed using a one-stage manual fibrometer method at dilutions ranging between 1:40 and 1:400 to obtain a measure of Factor VIII and Factor IX clotting activity. In vitro recovery of fibrinogen following reconstitution of both the control and heat-treated lyophilized fibrinogen samples was measured in a similar fashion. In some of the experiments, reconstituted plasma fractions were observed for light transmission at 580 nm in a Beckman Model 25 Spectrophotometer. Agarose gel electrophoresis with an ICL immunoelectrophoresis plate was carried out for several of the Factor VIII and Factor IX paired samples, using goat anti-human serum supplied by Hyland Diagnostics as a standard. The plates were specifically electrophoresed by a Buchler power supply set at 25 ma for 35 minutes. Upon completion of the electrophoresis, the plates were incubated in antisera for 18 to 24 hours and examined under indirect light. Panagell electrophoresis with a Worthington Diagnostics plate was carried out on additional paired samples of Factor VIII concentrate, using a Biorad water-cooled electrophoresis cell.

Further in vitro experiments were performed by heating lyophilized samples of Factor VIII concentrate in a water bath at room temperature and at predetermined temperatures for predetermined periods of time. Factor VIII_{Ag} was then determined using the method described by Laurell, "Electroimmuno Assay," The Scandinavian Journal of Clinical and Laboratory Investigation, Vo. 29, pp. 21-37 (1972). Factor VIII results were calculated for dilutions of 1:40, 1:80, 1:100 and 1:200 by plotting the height of the rockets of the Laurell standard curve against the percentage of dilution. Unknowns were expressed as a percentage of normal, based on the rocket heights of the unknowns in the standard curve.

In vivo recovery of clotting factor activity for both heat-treated Factor VIII and Factor IX concentrates was measured by injecting reconstituted, heat-treated lyophilized Factor VIII and Factor IX concentrates respectively into hemophilia A and hemophilia B dogs. A heat-treated, lyophilized Factor IX concentrate was also injected into a control dog. Laboratory parameters including Hct, serum protein, WBC, platelet count, blood smear, respiration rate, body temperature, pulse and clotting factor activity were subsequently ascertained for each of the animals at various intervals following the injections.

Results of the in vitro testing performed on Factor VIII concentrate are summarized in Tables I and II:

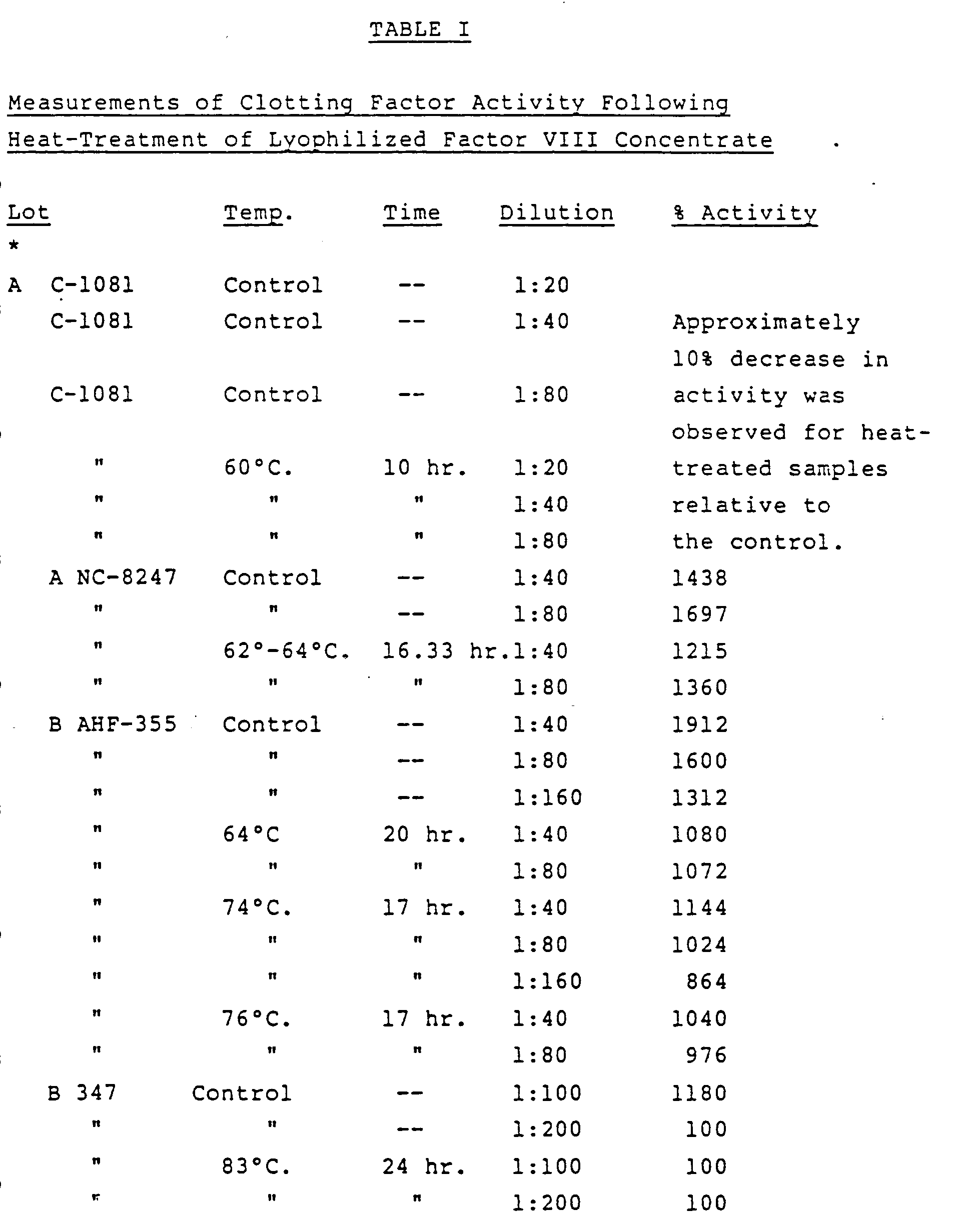

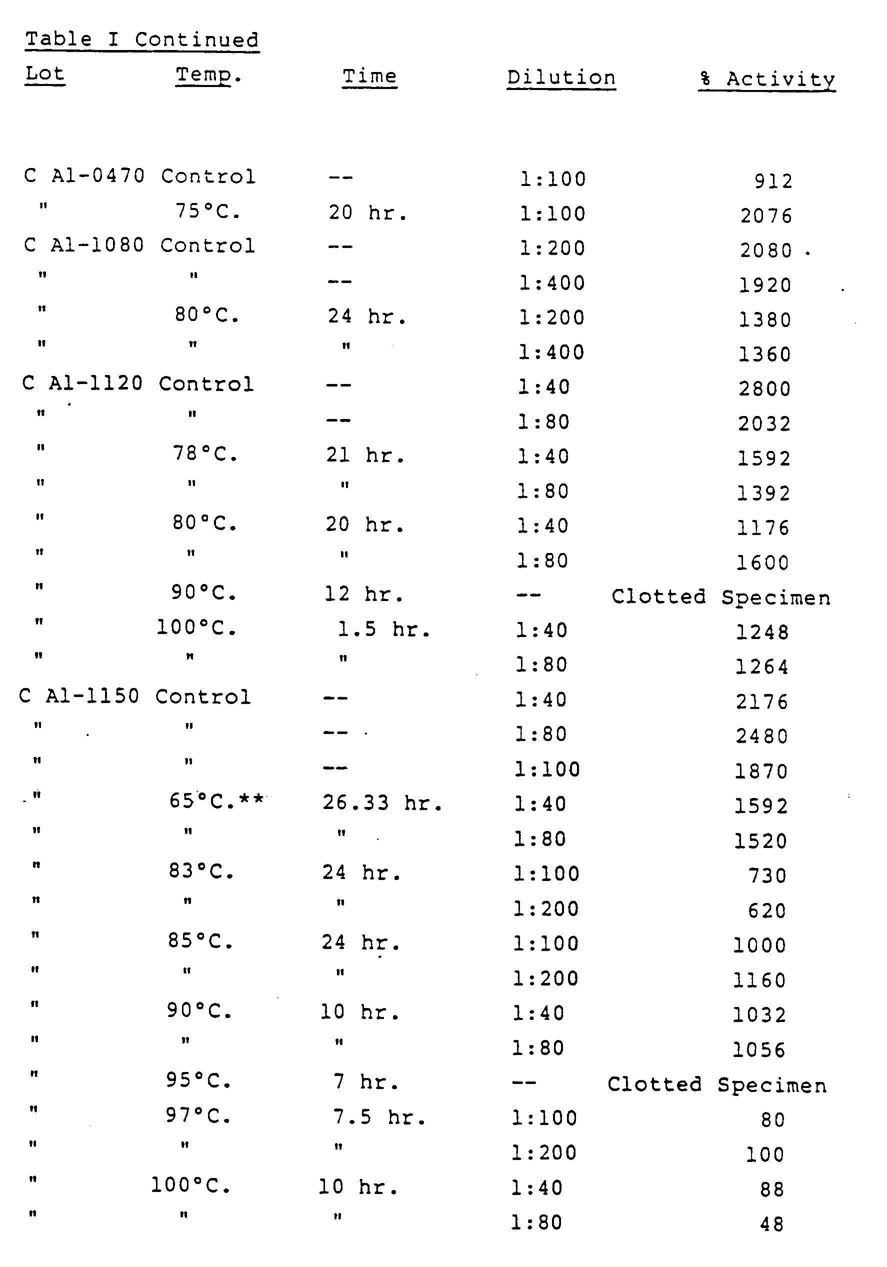

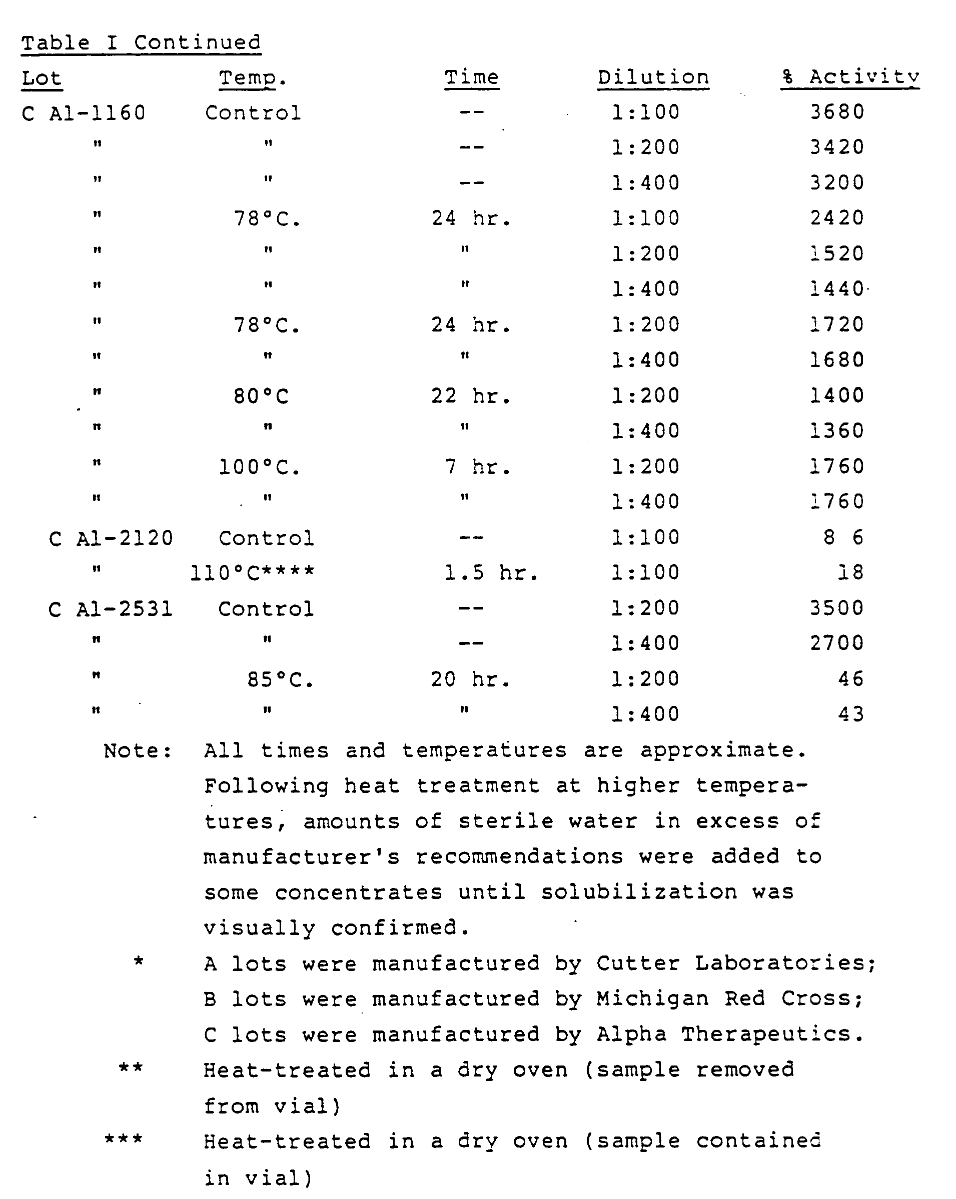

**TABLE II**

| Determination of Factor VIII_{Ag} Following Heat-Treatment of Lyophilized Factor VIII Concentrate | | | | | |
|---|---|---|---|---|---|
| Lot | Temp | Time | Dilution | Rocket Height (mm) | %Ag |
| B AHF-355 | Control | -- | 1:40 | 35 | 3720 |
| " | " | -- | 1:80 | 22 | 4080 |
| " | 64°C. | 20 hr. | 1:40 | 39 | 4240 |
| " | " | " | 1:80 | 26 | 5120 |
| " | 74°C. | 17 hr. | 1:40 | 40 | 4400 |
| " | " | " | 1:80 | 26 | 5120 |
| C Al-1120 | Control | -- | 1:100 | 15 | 4700 |
| " | 90°C. | 12 hr. | 1:100 | 0 | 0 |
| C Al-1150 | Control | -- | 1:200 | 13 | 7200 |
| " | 83°C. | 24 hr. | 1:200 | 12 | 6200 |
| " | 85°C. | 24 hr. | 1:200 | 15 | 9400 |
| " | 97°C. | 7.5 hr. | 1:200 | 0 | 0 |
| Note: All times and temperatures are approximate. Following heat treatment at higher temperatures, amounts of sterile water in excess of manufacturer's recommendations were added to some concentrates until solubilization was visually confirmed. * B lots were manufactured by Michigan Red Cross; C lots were manufactured by Alpha Therapeutics. | | | | | |

### Discussion of Selected Test Results

The results summarized in Tables I-II can be combined to provide a relative indication of clotting activity retention and recovery in lyophilized plasma fractions subjected to the heat-treatment process of the present invention. More particularly, the percentage activity or measured recovery at various dilutions of reconstituted Factor VIII can be averaged for individual control samples and compared with similarly-averaged percentage activity or measured recovery in corresponding paired samples of heat-treated Factor VIII concentrate. Where such comparisons are made, it can be seen that lyophilized Factor VIII concentrate obtained from one manufacturer (Lot No. A C-1081) and heated at 60° C. for 10 hours retained greated than 90% of its in vitro Factor VIII clotting activity in comparison to an unheated control. The reconstituted, heat-treated Factor VIII concentrate further exhibited an absorbance of 0.30 at 580 nm in comparison to an absorbance of 0.20 for the unheated control, and showed no differences relative to the unheated control following immunoelectrophoresis with the goat antihuman serum. Reconstituted Factor VIII concentrates from a different lot (Lot No. A NC-8747) of the same manufacturer, which had been heated in lyophilized form at 62° - 64° C. for approximately 16 hours and then stored at 6 C. for seven days, showed greater than 80% recovery of Factor VIII clotting activity in comparison to an unheated control. An overall increase in anodal migration relative to the unheated control was noted following immunoelectrophoresis against goat antihuman serum.

In similar fashion, lyophilized Factor VIII concentrate obtained from a second manufacturer (Lot No. C Al-1120), when heated at approximately 78° C. for 21 hours, showed 62% in vitro retention of clotting activity upon reconstitution as compared to an unheated control. Reconstituted Factor VIII concentrate from the same lot of the second manufacturer, after heat treatment in lyophilized form for 20 hours at approximately 80° C. still retained 57% in vitro clotting activity as compared to an unheated control, whereas reconstituted Factor VIII from the same lot of the second manufacturer, which had previously been heated in lyophilized form for one and one-half hour at approximately 100° C., retained approximately 52% in vitro clotting activity as compared to an unheated control. When a different lot (Lot No. C Al-1150) of lyophilized Factor VIII concentrate from the second manufacturer was heat-treated in accordance with the present invention, in vitro recoveries of clotting activity in comparison to the unheated control ranged from 67% for 26 hours and 20 minutes of heat treatment at 65° C. to 34% for 24 hours of heat treatment at 83° C. to 55% for 24 hours of heat treatment at 85° C. Measurements of Factor VIII antigen for the two samples of Factor VIII concentrate heat-treated at 83° C. and 85° C. respectively showed a 15% loss and no loss in antigen levels. It should also be noted that greatly improved solubilization of the latter sample of heat-treated Factor VIII concentrate was achieved by adding between 50 ml and 75 ml of sterile water to the sample rather than the 25 ml recommended by the manufacturer.

Heat treatment of lyophilized Factor VIII samples obtained from a third manufacturer (Lot No. AHF-355) confirmed results obserbed for the first two manufacturers. That is, heat treatment of the third manufacturer's lyophilized Factor VIII concentrate for 20 hours at 64° C. yielded clotting activity recovery of 61% in comparison to an unheated control, heat treatment of the same concentrate for 17 hours at 74° C. yielded clotting activity recovery of 63% and heat treatment of the same concentrate for 17 hours at 76° C. yielded clotting activity recovery of 57%. Factor VIII antigen levels in the reconstituted samples heated at 64° C. and 740° C showed no decrease when compared to the unheated control level.

As previously indicated, in vivo testing of heat-treated Factor VIII concentrates was carried out using hemophilia A or Factor VIII deficient dogs. The hemophilia A dog received reconstituted Factor VIII concentrate which had previously been heat-treated in lyophilized form at 60° C. for 10 hours. Results of the in vivo testing are reported in Table V below.

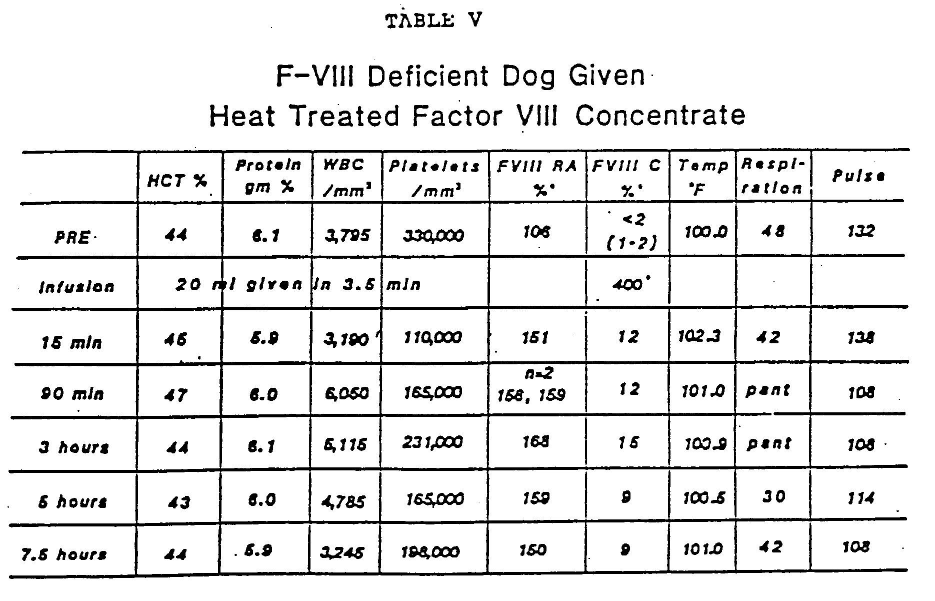

The results reported in Table V amply illustrate the marked increase in Factor VIII clotting activity for a hemophilia A dog following injection of heat-treated Factor VIII concentrate. The apparent absence of physiological stress or other adverse reaction as seen from the data in Tables V suggests that Factor VIII concentrates which have been processed according to the steps of the present invention remain biologically acceptable.

It has now been demonstrated that plasma fractions such as Factor VIII concentrates of varying purity can be safely heat-treated in lyophilized form at elevated temperatures for extended periods of time without significantly destroying the clotting activity of the concentrates. Visual obvervations confirm that the solubility of lyophilized Factor VIII concentrates is not deleteriously affected by heat-treatment inasmuch as the amount of sterile water added to the lyophilized concentrate samples during reconstitution can simply be increased until complete solubility is achieved.

It should, moreover, be apparent from extrapolation of the test results reported in Tables I-II that the method of the present invention can be performed at essentially any point during the plasma fractionation process. That is, at any point along the fractionation process where a plasma derivative can be lyophilized, heat treatment of the plasma derivative can be performed and the derivative resolubilized or reconstituted prior to continuation of the fractionation process. Thus, for example, where Factor VIII concentrate is ultimately derived from a plasma fractionation scheme such as that disclosed in Mammen, et al., "Treatment of Bleeding Disorders with Blood Components," Reviews of Hematology, Vol 1, p. 144 (1980), cryoprecipitate obtained from fresh frozen plasma, clarified extract obtained from cryoprecipitate and supernatant obtained from clarified extract can all be lyophilized and heat-treated in the same manner as the Factor VIII concentrate itself. Selection of an appropriate point in the plasma fractionation scheme for applying the heat treatment can then be based on pragmatic considerations such as cost or convenience.

Exemplary embodiments of the invention are the inactivation of the undesirable viruses i.e. cytomegalovirus and Epstein-Barr virus.

Furthermore different drying techniques could be employed to the plasma compositions such as plasma fractions, namely spray drying or vacuum drying.

The addition of sterile water to dry or lyophilized plasma in degrees greater than the volumes suggested by manufacturers creates plasma solutions of greater dilution than normally recommended; however, the effectiveness of the plasma and its constituents is not noticeably deteriorated. This is particularly useful where the temperatures to which the plasma composition is to be heated to non-activate undesirable viruses, is so high that clotting of the reconstituted plasma would normally occur unless the excess sterile water is added.

A particularly significant use of the process herein is the treatment of AHF-enriched compositions. Such compositions contain concentrations of AHF on a total protein weight basis that exceed those concentrations found in plasma or other conventional plasma protein fractions such as prothrombin complex (Factor IX concentrates), gamma globulin, albumin, fibrinogen or antithrobmin III. Such high AHF concentrations are needed to ensure that when the compositions are dissolved in water or other physiologically acceptable carrier that they can correct the patient's clotting abnormality sufficiently to demonstrate a favorable clincal effect, without requiring the infusion of excess water or extraneous proteins. Generally, a therapeutically effective dose of AHF can be determined readily by those skilled in the art. It will depend upon the clinical state of the patient, particularly the type of bleed encountered. Ordinarily, sufficient AHF should be infused to produce a patient plasma AHF level of at least 30% of that present in normal individuals, although for serious hemorrhages levels of 50% or more are preferred. This may be accomplished by infusion of a total number of AHF units as defined by the formula: $\text{Units required = body weight (kg)}$ $\text{x 0.4 x desired AHF increase (in % of normal)}$ .

The AHF concentration in the infusate should preferably not exceed 34 units/ml, but should be greater than 15 units/ml. If the concentration exceeds 34 units/ml, no more than 2 ml per minute should be infused. Infusated with less than 34 AHF units can be infused more rapidly, on the order of 10 to 20 ml over a 3 minute period. An AHF unit is defined as the activity of AHF present in 1 ml of normal pooled human plasma less than 1 hour old.

The AHF-enriched compositions contain greater than about 20 AHF units usually greater than about 300 AHF units/gram of protein. Obviously, the greater the AHF purity the more desirable the product. Compositions containing about from 100 to 2000 AHF units/ gram of protein are most feasible commercially.

The AHF compositions may be freed of other plasma proteins to varying degrees during the purification process, depending upon tee process used. Such plasma proteins include the blood clotting enzymes (herein meaning both the inactive proenzymes and the activated enzymes) such as prothrombin complex or Factor IX concentrate (Factors II, VII, IX and X), Factors XII or XIII, fibrinogen, albumin and gamma globulin. When an AHF composition is essentially free of blood clotting enzymes it contains subtherapeutic activities of the enzymes. The AHF compositions may be purified to a level in which the activity of any blood clotting enzyme is at a trace level, generally less than about 15 units/gram of protein, and preferably less than about 5 units/gram of protein. The ratio of AHF units to any individual enzyme unit activity in such compositions ordinarily ranges from 10:1 to 500:1, and is preferably greater than 300:1. A unit of enzyme activity for each of the various clotting enzymes in their activitated or proenzyme forms are defined in PCT International Application WO 82/03871 (published November 11, 1982).

The heat-treated AHF compositions herein are principally administered to patients having a congential deficiency of AHF. These patients fail to synthesize biologically active AHF, and are to be distinguished from those having acquired AHF inhibitors (probably antibodies) which also cause the symptoms of hemophilia. The latter group is preferrably treated with activated clotting enzyme compositions. The clotting enzyme compositions contain therapeutically effective amounts of the enzymes. However, the compositions herein generally will not contain such amounts of blood clotting enzymes, but instead essentially rely upon AHF activity for hemostasis.

The improved AHF compositions herein contain such lower quantities of denatured AHF than produced using prior processes. Generally, the amount of denatured AHF is less than about 50% of the total active and denatured AHF in the composition. Preferably it ranges about from 30% to 10%. The amount of denatured AHF is equivalent to the precent loss in AHF units after the virus-infected AHF composition is subjected to the heat treatment method described herein.

The effect of heating virus-contaminated AHF in the dry state may be followed by assaying the AHF clotting activity, as described above, and the viral titer. Where it is difficult to measure biologically active viral titer, as in the case of hepatitis viruses, candidate virus such as bacteriophage, sindbis, adenovirus or EHC virus may be employed as disclosed in PCT International Application WO 82/03871 (published November 11, 1982. A predetermined titer of the candidate virus is seeded into a solution of the composition to be heat treated, the solution lyophilized and various heat treatments undertaken. One selects the point at which actual measurements or regression analysis (statistical extrapolation) indicate the desired degree of viral inactivation as the conditions which will govern heat treatment of the product. These conditions generally will be the time and temperature of viral inactivation, although the moisture content of the composition will also be a variable that should be controlled. Time and temperature are described above. The moisture content should be below 5% by weight, ordinarily less than 1% by weight.

While some inactivation and denaturation of AHF takes place in the dry state heating process, it occurs at a lesser rate than the inactivation of even the more hardy candidate viruses or hepatitis virus. Thus, the infected AHF composition may be rendered substantially free of the virus in infectious form. This means that the titer of virus is reduced so low that infusion of therapeutic quantities of the product into a plurality of normal animal hosts for the virus will fail to produce clinical or serological evidence of infection in a host population, or will delay significantly the onset of infection in such population. Where an equivalent tissue culture assay is available to determine biological infectivity, it may be used in place of normal animal hosts as indicia of infectivity.

The mild dry state heating process herein preserves substantially all of the antigenicity of the infectious microbes, i.e. the epitopic sites on the organisms are not irreversibly denatured. This is in contrast to other methods involving covalent reactions with chemical substances, high energy irradiation or excessive heating.

The heating step may be accomplished in closed or open containers, as noted above, and is most efficaciously accomplished by simple, inexpensive techniques such as contact heating of the product containers, as in ovens or water or sand baths, or by infrared irradiation. For safety and for reasons of expense microwave heating is not preferred.

Several embodiments of the present invention have been illustrated hereinabove.

## Claims

1. A method for treating a substantially dry composition including human Factor VIII to reduce or eliminate the infectivity of cytomegalovirus or Epstein-Barr virus present in the composition, comprising heating the composition for a predetermined period of time at a temperature of 100 to 110°C to reduce or eliminate the infectivity of the virus.

2. A method for treating a substantially dry composition including human Factor VIII to reduce or eliminate the infectivity of cytomegalovirus or Epstein-Barr virus present in the composition, comprising heating the composition for a predetermined period of time at a temperature between 60°C and 125°C to reduce or eliminate the infectivity of said virus while retaining substantially all of the antigenicity of said virus.

3. The method according to claim 1 or 2, wherein said composition is Factor VIII concentrate.

4. An AHF enriched composition for the manufacture of a medicament agent for the treatment of bleeding disorders; said composition comprising a human Factor VIII concentrate essentially free of blood clotting enzymes and having been treated by heating for a predetermined period of time in the lyophilized form at a temperature between 60°C and 125°C, characterized by said human Factor VIII having both prior to and after heating an AHF purity of greater than about 300 AHF units/gram of protein, and by said composition being heated to render substantially inactive cytomegalovirus and said cytomegalovirus being rendered substantially inactive.

5. An AHF enriched composition for the manufacture of a medicament agent for the treatment of bleeding disorders; said composition comprising a human Factor VIII concentrate essentially free of blood clotting enzymes and having been treated by heating for a predetermined period of time in the lyophilized form at a temperature between 60°C and 125°C, characterized by said human Factor VIII having both prior to and after heating an AHF purity of greater than about 300 AHF units/grams of protein, and by said composition being heated to render substantially inactive an Epstein-Barr virus and said Epstein-Barr virus being rendered substantially inactive.

## Patentansprüche

1. Verfahren zur Behandlung einer im wesentlichen trockenen Zusammensetzung, die menschlichen Faktor VIII enthält, um die Infektiosität von Cytomegalovirus oder Epstein-Barr-Virus, der in der Zusammensetzung vorhanden ist, zu vermindern oder zu eliminieren, umfassend Erhitzen der Zusammensetzung über einen vorbestimmten Zeitraum bei einer Temperatur von 100 bis 110 °C, um die Infektiosität des Virus zu vermindern oder zu eliminieren.

2. Verfahren zur Behandlung einer im wesentlichen trockenen Zusammensetzung, die menschlichen Faktor VIII enthält, um die Infektiosität von Cytomegalovirus oder Epstein-Barr-Virus, der in der Zusammensetzung vorhanden ist, zu vermindern oder zu eliminieren, umfassend Erhitzen der Zusammensetzung über einen vorbestimmten Zeitraum bei einer Temperatur zwischen 60 °C und 125 °C umfaßt, um die Infektiosität des Virus zu vermindern oder zu eliminieren, wobei im wesentlichen die ganze Antigenität des Virus erhalten bleibt.

3. Verfahren nach Anspruch 1 oder 2, wobei die Zusammensetzung Faktor VIII-Konzentrat ist.

4. AHF-angereicherte Zusammensetzung zur Herstellung eines Arzneimittels für die Behandlung von Blutungskrankheiten; wobei die Zusammensetzung, die ein im wesentlichen von Blutgerinnungsenzymen freies Konzentrat des menschlichen Faktors VIII enthält und die durch Erhitzen über einen vorbestimmten Zeitraum in der lyophilisierten Form bei einer Temperatur zwischen 60 °C und 125 °C behandelt worden ist, dadurch gekennzeichnet ist, daß der menschliche Faktor VIII vor und nach dem Erhitzen eine AHF-Reinheit von mehr als etwa 300 AHF-Einheiten/Gramm Protein hat, und daß die Zusammensetzung erhitzt worden ist, um Cytomegalovirus zu inaktivieren, und daß der Cytomegalovirus im wesentlichen inaktiviert worden ist.

5. AHF-angereicherte Zusammensetzung zur Herstellung eines Arzneimittels für die Behandlung von Blutungskrankheiten; wobei die Zusammensetzung, die ein im wesentlichen von Blutgerinnungsenzymen freies Konzentrat des menschlichen Faktors VIII enthält und durch Erhitzen über einen vorbestimmten Zeitraum in der lyophilisierten Form bei einer Temperatur zwischen 60 °C und 125 °C vorbehandelt worden ist, dadurch gekennzeichnet ist, daß der menschliche Faktor VIII vor und nach dem Erhitzen eine AHF-Reinheit von mehr als etwa 300 AHF-Einheiten/Gramm Protein hat und daß die Zusammensetzung erhitzt worden ist, um einen Epstein-Barr-Virus im wesentlichen zu inaktivieren, und daß der Epstein-Barr-Virus im wesentlichen inaktiviert worden ist.

## Revendications

1. Procédé pour traiter une composition pratiquement sèche contenant du Facteur VIII humain pour réduire ou éliminer l'infectivité du cytomegalovirus ou du virus d'Epstein-Barr présent dans la composition, comprenant le chauffage de la composition pendant un temps déterminé, à une température de 100 à 110°C, pour réduire ou éliminer l'infectivité du virus.

2. Procédé de traitement d'une composition pratiquement sèche contenant du Facteur VIII humain pour réduire ou éliminer l'infectivité du cytomegalovirus ou du virus d'Epstein-Barr présent dans la composition, comprenant le chauffage de la composition pendant un temps déterminé, à une température comprise entre 60°C et 125°C, pour réduire ou éliminer l'infectivité de ce virus tout en conservant la quasi-totalité de l'antigénicité de ce virus.

3. Procédé selon les revendications 1 ou 2, dans lequel cette composition est un concentré de Facteur VIII.

4. Composition enrichie en AHF pour la fabrication d'un agent médicamenteux pour le traitement des troubles hémorragiques; cette composition comprenant un concentré de Facteur VIII humain pratiquement exempt d'enzymes de la coagulation du sang et ayant été traitée par chauffage pendant un temps déterminé, sous forme lyophilisée, à une température comprise entre 60°C et 125°C, caractérisée en ce que ce Facteur VIII humain présente avant et après le chauffage une pureté de l'AHF supérieure à environ 300 unités de AHF/gramme de protéine, et en ce que cette composition est chauffée pour inactiver pratiquement le cytomegalovirus, et en ce que ce cytomegalovirus est rendu pratiquement inactif.

5. Composition enrichie en AHF pour la fabrication d'un agent médicamenteux pour le traitement des troubles hémorragiques; cette composition comprenant un concentré de Facteur VIII humain pratiquement exempt d'enzymes de la coagulation du sang et ayant été traitée par chauffage pendant un temps déterminé, sous la forme lyophilisée, à une température comprise entre 60°C et 125°C, caractérisée en ce que ce Facteur VIII humain présente avant et après le chauffage une pureté de l'AHF supérieure à environ 300 unités d'AHF/gramme de protéine, et en ce que cette composition est chauffée pour inactiver pratiquement le virus d'Epstein-Barr, et en ce que ce virus d'Epstein-Barr est rendu pratiquement inactif.
